# EUROPÄISCHE PATENTANMELDUNG

(11) **EP 1 195 381 A1**
(43) Veröffentlichungstag der Anmeldung: **10.04.2002**
(21) Anmeldenummer: 00121138.2
(22) Anmeldetag: 28.09.2000
(51) Int. Cl.: C07K 14/18, C12N 15/51, A61K 39/29, G01N 33/68, A61P 31/14

(54) **CD4+ T-Lymphozyten spezifische Hepatitis C Virus-Epitope**

(71) Anmelder: Immusystems GmbH, 80538 München (DE)
(72) Erfinder: Gerlach, Jörn, Tilman, Dr., 81479 München (DE); Diepolder, Helmut, Dr., 80689 München (DE)
(74) Vertreter: Grünecker, Kinkeldey, Stockmair & Schwanhäusser Anwaltssozietät

(57) **Zusammenfassung**

Die Erfindung betrifft Hepatitis C Virus-Epitope, die gegenüber CD4⁺ T-Lymphozyten spezifisch sind, sowie Impfstoffe, die diese Epitope enthalten.

SEQUENZPROTOKOLL

<110> Immusystems

<120> CD4+-Lymphozyten spezifische HCV-Epitope

<130> DA1Ger

<140>
<141>

<160> 6

<170> PatentIn Ver. 2.1

<210> 1
<211> 10
<212> PRT
<213> Hepatitis C virus

<400> 1
Tyr Leu Val Ala Tyr Gln Ala Thr Val Cys
  1               5                  10

<210> 2
<211> 21
<212> PRT
<213> Hepatitis C virus

<400> 2
Val Val Thr Ser Thr Trp Val Leu Val Gly Gly Val Leu Ala Ala Leu
  1               5                  10                  15

Ala Ala Tyr Cys Leu
             20

<210> 3
<211> 11
<212> PRT
<213> Hepatitis C virus

<400> 3
Gln Tyr Leu Ala Gly Leu Ser Thr Leu Pro Gly
  1               5                  10

<210> 4
<211> 9
<212> PRT
<213> Hepatitis C virus

<400> 4
Ile Ala Ser Leu Met Ala Phe Thr Ala
  1               5

<210> 5
<211> 9
<212> PRT
<213> Hepatitis C virus

<400> 5
Phe Asn Ile Leu Gly Gly Trp Val Ala
1               5

<210> 6
<211> 20
<212> PRT
<213> Hepatitis C virus

<400> 6
Ser Pro Val Phe Thr Asp Asn Ser Ser Pro Pro Ala Val Pro Gln Ser
1               5                   10                  15

Phe Gln Val Ala
            20

## Beschreibung

Die Erfindung betrifft Hepatitis C Virus-Epitope, die gegenüber CD4⁺ T-Lymphozyten spezifisch sind, sowie Impfstoffe, die diese Epitope enthalten.

Das Hepatitis C Virus, im nachstehenden HCV genannt, wurde 1989 identifiziert und ist ein RNA Virus aus der Familie der Flaviviridae. Es besteht aus einem RNA-Einzelstrang von ca. 9400 Nukleotiden, die ein ca. 3000 Aminosäuren langes Vorläuferpolyprotein kodieren. Dieses Polyprotein wird in einem offenen Leserahmen translatiert und posttranslationell proteolytisch gespalten. Das Virus ist hochvariabel, und es existieren verschiedene Virusisolate, die als Genotypen bezeichnet werden und deren geographische Verteilung sehr unterschiedlich ist. Mehr als sechs Genotypen werden heute weltweit unterschieden. Diese Genotypen wiederum werden in Subtypen unterteilt. Die genetische Variabilität besteht interindividuell und intraindividuell (innerhalb eines infizierten Individuums). Die intraindividuellen Subtypen sind die sogenannten HCV Quasispezies, verwandte aber unterschiedliche Virussequenzen, die bei ungenauer Replikation entstehen.

Mit einer Prävalenz von ca. ein bis drei Prozent weltweit ist Hepatitis C eine der bedeutendsten chronischen Virusinfektionen. Man geht derzeit von mindestens 180 Mio. infizierten Individuen aus. Nach Berechnungen des Center of Disease Control in den USA wird es aufgrund der langen Latenzzeit nach der Infektion mit dem HCV außerdem noch zu einem Anstieg der Hepatitis C assoziierten Erkrankungen bis zum Jahre 2010 kommen.

Das HCV wird überwiegend parenteral übertragen und war bis zu seiner Entdeckung die Hauptursache für die Posttransfusionshepatitis NonA-NonB. Durch routinemäßiges Testen aller Blutprodukte mit HCV-Antikörpertests der 2. und 3. Generation hat die Zahl der Posttransfusionshepatitiden drastisch abgenommen. Die sogenannte sporadische Hepatitis C sowie iv.-Drogenmißbrauch gelten heute als Hauptübertragungswege neuer HCV Infektionen. Es sind derzeit keine Maßnahmen bekannt, um Neuinfektionen auf diesen Wegen wirksam zu verhindern.

Das HCV verursacht eine chronische Leberentzündung (Hepatitis), die im langjährigem Verlauf zu weiteren Komplikationen, wie einer Leberzirrhose, führen kann. Im Rahmen einer jahrelang bestehenden Leberzirrhose kommt es bei ca. 5% aller Infizierten zur Entwicklung eines hepatozellulären Karzinoms. In der westlichen Welt nimmt die Hepatitis C deshalb den ersten Rang als Ursache einer Lebertransplantation ein. Die Kosten für das Gesundheitswesen durch diese Transplantationen sind erheblich.

Bei chronischer Hepatitis C sind zwar gegen fast alle Virusproteine Antikörper nachweisbar, es gibt im Gegensatz zur Hepatitis B jedoch keine Anti-HCV-Antikörperkonstellation, die eine Immunität gegenüber HCV oder eine Ausheilung anzeigt. Auch die Präsenz von Antikörpern gegen das HCV während einer chronischen HCV Infektion mildert den Verlauf nicht. Im Gegenteil scheint eine erfolgreiche Therapie mit einem Absinken der Antikörpertiter verbunden zu sein. Daher ist es nicht möglich, eine Infektion mit Hepatitis C durch eine konventionelle, prophylaktische Impfung mit Hüllprotein, wie sie bei der Hepatitis B erfolgreich durchgeführt wird, zu verhindern. Eine therapeutische Impfung ist daher derzeit nicht verfügbar.

Die einzige derzeit zugelassene Therapie ist eine Behandlung mit Interferon alpha allein oder in Kombination mit Ribavirin für 3 bis 12 Monate. Diese Therapieform ist sehr kostenintensiv, häufig mit Nebenwirkungen belastet und führt nur in ca. 40% der Fälle zu einer dauerhaften Viruselimination.

Aus Journal of Virology, Band 71, Seiten 6011 bis 6019 und Hepatology, Band 30, Nr. 4, 1999, Seiten 1088 bis 1098 ist bekannt, daß durch direkte periphere Blut-T-Zellstimulation und Spezifitätsanalyse von HCV-spezifischen T-Zelllinien, hochimmunogene T-Zell-Epitope innerhalb der Core, NS3- und NS4-Region des Hepatitis C Virus identifiziert werden können. Dabei werden aus peripherem Blut T-Lymphozyten isoliert und die darin enthaltenen HCV spezifischen CD4+ T-Lymphozyten durch wiederholte Stimulation mit dem entsprechenden Virusprotein in vitro zu sogenannten spezifischen T-Zelllinien angereichert. Durch die Analyse des Wachstumsverhaltens (Einbau radioaktiv markierter Nukleotide) dieser spezifischen T-Zelllinien nach Stimulation mit HCV-Protein und/oder den kleineren Untereinheiten, den Peptiden, kann die Sequenz des T-Zell-Epitops eingeengt werden. Insbesondere die Analyse von T-Zelllinien ergibt im Vergleich zu T-Zell-Klonen aufgrund des Zellgemisches ungenaue Ergebnisse hinsichtlich der von den CD4+ T-Lymphozyten spezifisch erkannten Sequenz.

Daher liegt der vorliegenden Erfindung die Aufgabe zugrunde, gegenüber CD4⁺ T-Lymphozyten spezifische HCV-Epitope zu isolieren und die durch den CD4+ T-Lymphozyten identifizierten HCV-Epitope für einen Impfstoff zur Prophylaxe und/oder Therapie einer HCV-Infektion zur Verfügung zu stellen.

Die Lösung der Aufgabe sind die nachstehend genannten gegenüber CD4⁺ T-Lymphozyten spezifischen HCV-Epitope, enthalten die Sequenz:
1) YLVAYQATVC;
2) VVTSTWVLVGGVLAALAAYCL;
3) QYLAGLSTLPG;
4) IASLMAFTA;
5) FNILGGWVA; und
6) SPVFTDNSSPPAVPQSFQVA
und Derivate davon mit vergleichbarer Spezifität.

Eine weitere Lösung ist ein Impfstoff, der mindestens eines der erfindungsgemäßen Epitope enthält.

Die Erfindung wird mittels der Figuren verdeutlicht. Es zeigen
- Fig. 1: die Epitopenkartierung des Epitops an der Position aa1765 - 1784;
- Fig. 2: das Kartieren des Epitops an der Position aa1773 - 1783 mittels N- oder C-terminal trunkierter Peptide;
- Fig. 3: die Epitopenkartierung des Epitops an der Position aa1785 - 1804;
- Fig. 4: das Kartieren des Epitops an der Position aa1787 - 1795 mittels N- oder C-terminal trunkierter Peptide;
- Fig. 5: die Epitopenkartierung des Epitops an der Position aa1655 - 1674; und
- Fig. 6: das Kartieren des Epitops an der Position aa1585 - 1594 mittels N- oder C-terminal trunkierter Peptide.

Die erfindungsgemäßen Epitope werden spezifisch von CD4⁺T-Lymphozyten erkannt, die bei einer selbstlimitierten HCV-Infektion im Patienten gebildet werden.

Die Lage der erfindungsgemäßen Epitope auf dem HCV-Protein ist jeweils für 1) aa1585 - 1594, 2) aa1655 - 1675, 3) aa1773 - 1783, 4) aa1787 - 1795, 5) aa1809 - 1817 und 6) aa1207 - 1226, wobei sich die angegebenen Positionen beispielsweise auf die Veröffentlichung CHOO, Q.-L. et al. PNAS 1991 beziehen.

Die erfindungsgemäßen Epitope, enthaltend die vorstehend genannten Sequenzen, sind vorzugsweise die Sequenzen selbst.

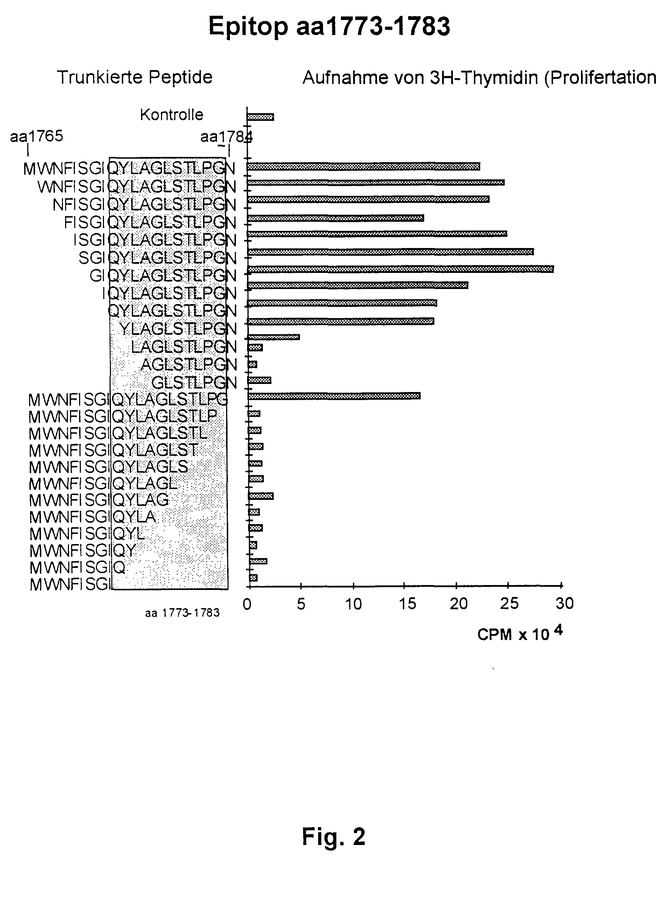

Zur Identifizierung dieser gegenüber CD4⁺ T-Lymphozyten spezifischen HCV-Epitope wurden bei Patienten mit akuter Hepatitis C Infektion, die einen selbstlimitierten Verlauf der Erkrankung hatten und bei denen gleichzeitig in vitro eine starke HCV-spezifische T-Lymphozytenaktivität nachgewiesen werden konnte, T-Zellklone dieser virusspezifischen T-Lymphozyten aus T-Zelllinien mit Hilfe der klassischen Grenzverdünnungsmethode isoliert und kloniert. Dieses Verfahren gestattet die Multiplikation einer einzelnen Zelle um viele Zehnerpotenzen und ermöglicht die Charakterisierung der Antigenspezifität mit einer klonalen Zellpopulation. Mit Hilfe der HCV-spezifischen T-Zellklone erfolgte eine genaue Charakterisierung des jeweils erkannten Epitops mittels N-terminal und C-terminal trunkierter Peptide, wie in den Fig. 2, 4 und 6 gezeigt.

Die erfindungsgemäßen Derivate der Epitope 1) bis 6) mit vergleichbarer Spezifität können in derselben Weise wie diese Epitope unter Verwendung der HCV-spezifischen T-Zellklone mittels N- und C-terminalen trunkierter Peptide gefunden werden oder durch Austausch einzelner oder mehrerer Aminosäuren in den Sequenzen 1) bis 6) und Überprüfen der Spezifität dieser geänderten Sequenzen.

Die erfindungsgemäßen Epitope sind hochimmunogene, hochkonservierte und in unmittelbarer Nachbarschaft zu bekannten, gegenüber CD8+ T-Lymphozyten spezifischen HCV-Epitopen gelegene Sequenzen des HCVs.

Als gegenüber CD4⁺ T-Lymphozyten spezifische HCV-Epitope können diese neben der Induktion von CD4+ T-Lymphozyten auch sog. T-Zell-Hilfe für zytotoxische CD8+ T-Lymphozyten vermitteln. Diese CD8+-T-Lymphozyten werden durch die Zytokine stimulierter CD4+ T-Lymphozyten aktiviert. Besonders wichtig ist in diesem Zusammenhang auch die unmittelbare Nachbarschaft der hier gefundenen HCV-Epitope zu bekannten CD8+ Epitopen.

Die Epitope 1) bis 5) wurden durch frische periphere mononukleäre Zellen (PBMC) von 16 Patienten mit akuter HCV erkannt, nämlich für 1) von 6 Patienten, für 2) von 4 Patienten, für 3) von 6 Patienten, für 4) von 3 Patienten und für 5) von 4 Patienten.

Die erfindungsgemäßen Epitope stimulierten nicht nur die T-Lymphozyten der Patienten, von denen die T-Zellklone stammen, sondern auch die frischen peripheren mononukleären Zellen (PBMC) unterschiedlicher Patienten mit akuter Hepatitis C und selbstlimitiertem Verlauf. Das für die Antigenpräsentation bedeutsame HLA (Klasse I und II) dieser Personen mit positiver Reaktion auf diese Epitope war unterschiedlich, so daß von einer gewissen Promiskuität der Peptide (Präsentation auf unterschiedlichen HLA-Rezeptoren) ausgegangen werden kann. Somit sind sie im Falle einer protektiven T-Zellimpfung hervorragend zur Impfung von gesunden Menschen oder Hepatitis C-Patienten mit jeweils unterschiedlichen HLA-Merkmalen geeignet.

Die erfindungsgemäßen Epitope können allein oder mit einem oder mehreren Hilfsstoffen als Arzneimittel, vorzugsweise als Impfstoff, eingesetzt werden. Der erfindungsgemäße Impfstoff enthält mindestens ein erfindungsgemäßes Epitop, vorzugsweise ein Gemisch aus erfindungsgemäßen Epitopen, insbesondere das Epitop an der Position aa1773 - 1783, dem gegebenenfalls weitere erfindungsgemäße Epitope oder Epitope an anderen Positionen zugesetzt werden können.

Die Hilfsstoffe werden vorzugsweise ausgewählt aus der Gruppe, bestehend aus fowlpoxvirus, modifiziertes Vakziniavirus Ankara, Virosomen, Transvax® und anderen die Immunreaktion verstärkenden Stoffen.

Der erfindungsgemäße Impfstoff kann oral, parenteral, intramuskulär, intravenös, subcutan oder intracutan verabreicht werden.

Bei den erfindungsgemäßen Epitopen handelt es sich um Epitope, die als T-Zellstimulierender Impfstoff eingesetzt werden können. Ein Impfstoff, der die erfindungsgemäßen Epitope enthält, hat gegenüber einer Impfung mit dem gesamten Virusprotein, das verschiedenste Epitope für virusspezifische T-Lymphozyten enthält und nur B-Lymphozyten und CD4⁺T-Lymphozyten induziert, den Vorteil, dass es spezifische T-Lymphozyten, CD4⁺- und/oder CD8⁺-T-Lymphozyten selektiv induziert. Außerdem werden dadurch antagonistische Effekte bzw. die Gefahr von iatrogen erzeugten Autoimmunreaktionen, die bei Impfungen mit ganzen Proteinen auftreten können, vermieden. Die erfindungsgemäßen Epitope haben zusätzlich eine höhere Immunogenität im Vergleich zu dem gesamten Virusprotein, wodurch ein besseres Impfergebnis erreicht wird.

Der erfindungsgemäße Impfstoff ermöglicht somit im gesunden Menschen die Induktion einer Immunantwort und dient daher als prophylaktische Impfung. Auch bei chronisch HCV-infizierten Menschen kann der erfindungsgemäße Impfstoff eine Immunantwort induzieren und somit als therapeutischer Impfstoff dienen.

Die kodierende c-DNA dieser Epitope kann in einem DNA-Impfstoff, einer speziellen Impfmethode, eingesetzt werden. Dabei wird die für die entsprechenden Epitope codierende DNA in einen Vector kloniert. Dieses Konstrukt wird wiederum dem zu impfenden Individuum parenteral verabreicht (z.B. Imunology and Cell Biology, Band 75, Seite 382 bis 388). Entsprechend des degenerierten genetischen Codes können verschiedene DNA-Sequenzen eines der erfindungsgemäßen Epitope kodieren (siehe Current protocols, Wiley).

Die erfindungsgemäßen Epitope können auch in der Diagnose des Verlaufs einer HCV-Infektion Verwendung finden, indem die Menge an CD4⁺ T-Lymphozyten, die spezifisch das betreffende Epitop erkennen, im Blut des Patienten mit einer Hepatitis C Infektion überwacht wird. Dies kann beispielsweise mit einem diagnostischen Kit durchgeführt werden, der eines oder mehrere der erfindungsgemäßen Epitope umfaßt.

### Beispiel 1

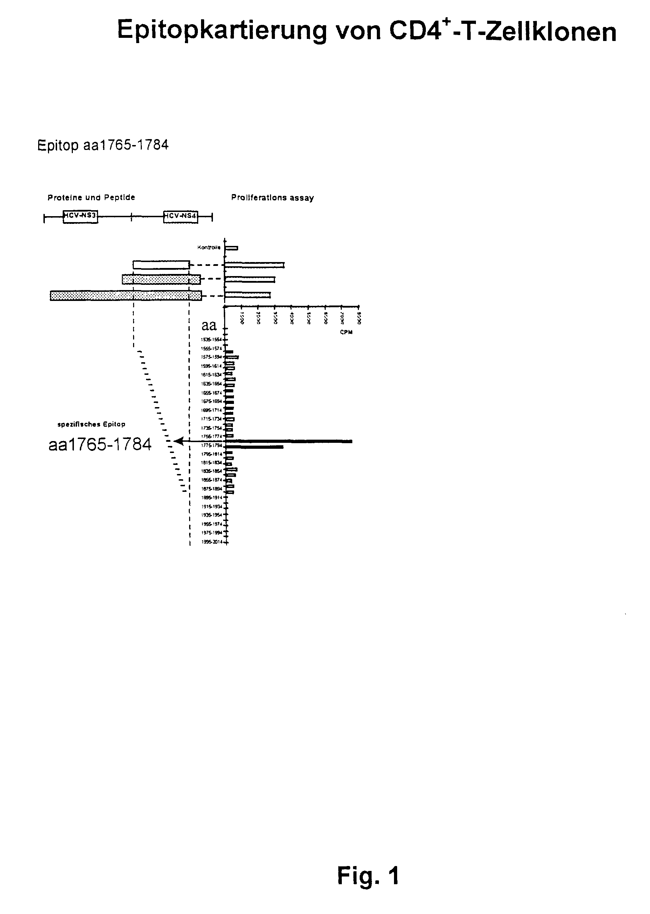

Von Patienten mit selbstlimitiertem Verlauf einer akuten Hepatitis C wurde in den ersten 6 Monaten nach Erkrankungsbeginn heparinisiertes Blut abgenommen. Durch Dichtegradientenzentrifugation auf Ficollgradienten wurden die frischen peripheren mononukleären Blutzellen (PBMC) isoliert und in einem Kulturmedium (RPMI1640, Gibco) suspendiert. 50 µl dieser Zellsuspension (Konzentration von 1x10⁶ Zellen pro ml) wurde auf sterile 96 Loch-Kulturplatten verbracht. Jeweils zehn Proben dieses Zellgemisches wurden durch Zugabe eines rekombinanten HCV-Proteins stimuliert. Die Endkonzentration des Proteins betrug 1µg/ml. Die Zellkulturplatten wurden über 5 Tage bei 37°C und 5%CO₂ kultiviert. Am Tag 6 wurde der Kultur IL-2 zugegeben. Die Endkonzentration von IL-2 betrug 15U/ml und die Zellen wurden für weitere drei Tage bei 37°C und 5%CO₂ kultiviert. Diejenigen Löcher der Kulturplatte, die am Tag zehn bei mikroskopischer Kontrolle stark stimuliert erschienen, wurden eingesammelt und in seriellen Verdünnungsstufen auf eine weitere Kulturplatte verteilt. Mikroskopisch wurde ein Loch ausgewählt, welches annähernd 150 Zellen enthielt. Diese 150 Zellen wurden mit Medium verdünnt und auf 300 Löcher verteilt, so daß statistisch eine halbe Zelle in jedem Loch enthalten war. Sodann wurde IL-2 bis zu einer Endkonzentration von 15U/ml zugesetzt. Zusätzlich wurden 3x10⁴ autologe und mit 3000 Rad bestrahlte PBMC sowie Phythaemagglutinin (PHA) als Wachstumsfaktor zu jedem Loch zugegeben. Die Klone wurden expandiert (vermehrt) und anschließend in einem Proliferationsassay mit HCV-Proteinen auf Antigenspezifität geprüft. Die Antigenspezifität wurde zunächst durch Stimulation mit Proteinen und sodann, im Falle eines positiven Ergebnisses, mit 20 Aminosäuren langen Peptiden (20mer-Peptiden) bestimmt, wie in Figur 1 gezeigt.

Dazu wurden T-Zellklone mit Proteinen, wie in Figur 1 links dargestellt, und antigenpräsentierenden Zellen stimuliert, und die Stimulation wurde als Einbau radioaktiv markierten ³H, wie in Figur 1 rechts dargestellt, gemessen. Zur weiteren Einengung des spezifisch erkannten Epitops wurde mit zu der Proteinsequenz korrespondierenden 20 mer-Peptiden, die jeweils um 10 Aminosäuren überlappen, stimuliert und der Einbau des ³H erneut gemessen. So wurde das entsprechende Epitop auf 20 Aminosäuren eingeengt.

Das Epitop, die kleinste noch stimulierende Sequenz innerhalb des 20mer Peptides, wurde mit trunkierten Peptiden analysiert, d.h. das ursprüngliche 20mer Peptid wurde in aufsteigender Reihe von N-terminal gekürzt, wie in Figur 2 gezeigt. Ebenso wurde mit Peptiden, die von C-terminal trunkiert waren, verfahren. Ein Proliferationsassay zeigte, bis zu welcher Aminosäure das Peptid gekürzt werden konnte, ohne nennenswerte Stimulationsverluste hinnehmen zu müssen.
Man erhält das Epitop QYLAGLSTLPG, wie in Figur 2 gezeigt. Durch Sequenzvergleich der charakterisierten Epitope mit Sequenzen der bekannten Genotypen ganzer HCV-Proteine stellte sich trotz der bekannt hohen genetischen Variabilität des HCV ein hoher Konservierungsgrad der Epitope heraus.

### Beispiel 2

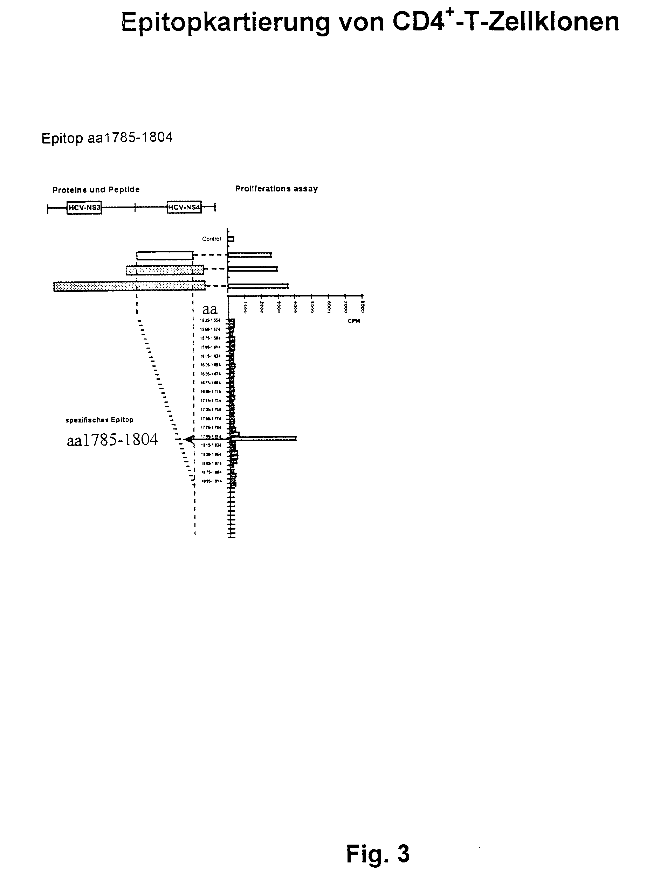

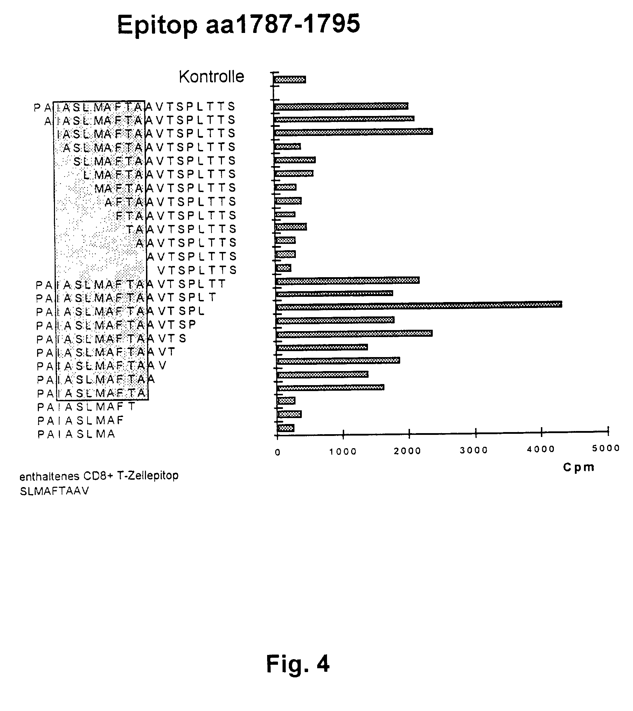

Gemäß dem Verfahren nach Beispiel 1 wurden Klone erhalten. Die Antigenspezifität dieser Klone wurde dann, wie in Figur 3 gezeigt, bestimmt. Man erhält das entsprechende Epitop mit 20 Aminosäuren. Das Epitop innerhalb dieses 20mer Peptids wurde sodann, wie in Figur 4 gezeigt, bestimmt. Man erhält das Epitop IASLMAFTA.

### Beispiel 3

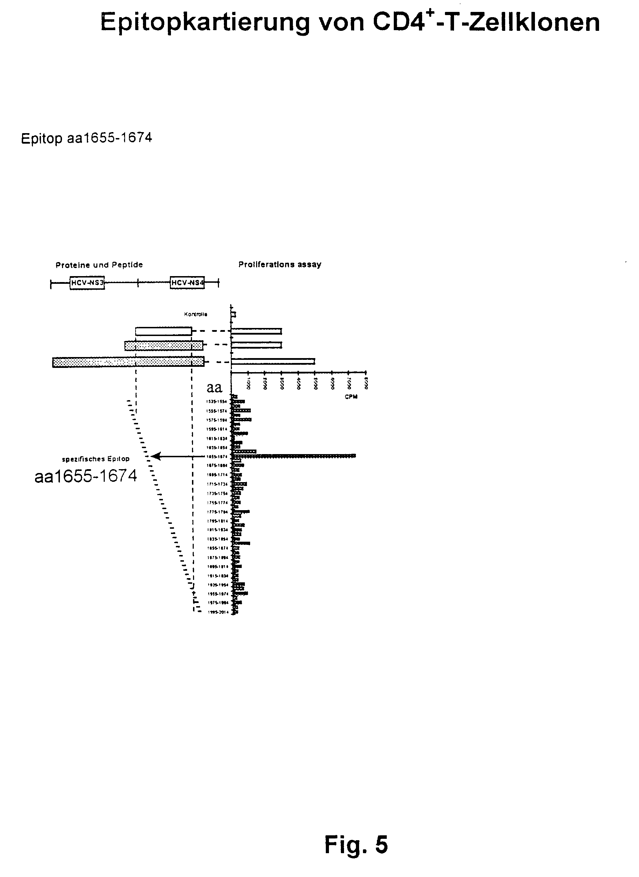

Gemäß dem Verfahren nach Beispiel 1 wurden Klone erhalten. Die Antigenspezifität dieser Klone wurde dann, wie in Figur 5 gezeigt, bestimmt. Man erhält das entsprechende Epitop mit 20 Aminosäuren, nämlich VVTSTWVLVGGVLAALAAYCL, gemäß Beispiel 1.

### Beispiel 4

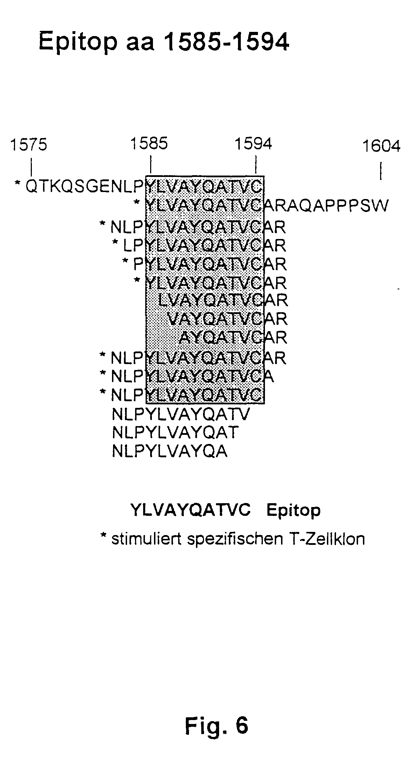

Gemäß dem Verfahren nach Beispiel 1 wurden Klone erhalten. Die Antigenspezifität wurde ebenfalls, wie in Beispiel 1 beschrieben, bestimmt und das entsprechende Epitop mit 20 Aminosäuren erhalten. Wie in Figur 6 gezeigt, wurde sodann das Epitop bestimmt, nämlich YLVAYQATVC.

## Patentansprüche

1. CD4⁺ T-Lymphozyten spezifische HCV-Epitope, enthaltend die Sequenz:
1) YLVAYQATVC;
2) VVTSTVWLVGGVLAALAAYCL;
3) QYLAGLSTLPG;
4) IASLMAFTA;
5) FNILGGWVA, und
6) SPVFTDNSSPPAVPQSFQVA
und Derivate hiervon mit vergleichbarer Spezifität.

2. Impfstoff, mindestens ein HCV-Epitop nach Anspruch 1 umfassend.

3. Impfstoff nach Anspruch 2, zusätzlich mindestens einen Hilfsstoff, ausgewählt aus der Gruppe, bestehend aus fowlpoxvirus, modifiziertem Vakziniavirus Ankara, Virosomen, Transvax und anderen die Immunreaktion verstärkenden Stoffen, umfassend.

4. Impfstoff nach Anspruch 2 oder 3, in Form einer Injektionslösung.

5. HCV-Epitop nach Anspruch 1 als Arzneimittel.

6. Verwendung mindestens eines HCV-Epitops nach Anspruch 1 zur Herstellung einer Zusammensetzung zur Prophylaxe und/oder Therapie einer Hepatitis C Infektion.

7. Verwendung mindestens eines HCV-Epitops nach Anspruch 1 in der Diagnose einer Hepatitis C Infektion.

8. Diagnostischer Kit, mindestens eines der Epitope nach Anspruch 1 umfassend.

9. c-DNA, eines der HCV-Epitope nach Anspruch 1 kodierend.

10. Impfstoff, mindestens eine c-DNA nach Anspruch 9 umfassend.
